Europäisches Patentamt

European Patent Office

Office européen des brevets

(19)

(11) **EP 0 907 612 B1**

(12) **FASCICULE DE BREVET EUROPEEN**

(45) Date de publication et mention
de la délivrance du brevet:
**21.08.2002  Bulletin 2002/34**

(21) Numéro de dépôt: **97927232.5**

(22) Date de dépôt: **04.06.1997**

(51) Int Cl.[7]: $C01B\ 33/193$, $A61K\ 7/16$

(86) Numéro de dépôt international:
**PCT/FR97/00987**

(87) Numéro de publication internationale:
**WO 97/046485 (11.12.1997 Gazette 1997/53)**

(54) **SILICE SUSCEPTIBLE D'ETRE UTILISEE DANS LES COMPOSITIONS DENTIFRICES**

ZUR VERWENDUNG IN ZAHNPASTEN GEEIGNETE KIESELSÄURE

SILICA CAPABLE OF BEING USED IN TOOTHPASTE COMPOSITIONS

(84) Etats contractants désignés:
**AT BE CH DE DK ES FI FR GB GR IE IT LI NL PT
SE**

(30) Priorité: **06.06.1996  FR 9607187**

(43) Date de publication de la demande:
**14.04.1999  Bulletin 1999/15**

(73) Titulaire: **RHODIA CHIMIE
92408 Courbevoie Cédex (FR)**

(72) Inventeurs:
  • **AMICHE, Frédéric
    F-92420 Vaucresson (FR)**
  • **DROMARD, Adrien
    F-69006 Lyon (FR)**

(74) Mandataire: **Fabre, Madeleine-France et al
Rhodia Services,
Direction de la Propriété Industrielle,
40, rue de la Haie-Coq
93306 Aubervilliers Cedex (FR)**

(56) Documents cités:
    **EP-A- 0 064 620          WO-A-95/18066
    FR-A- 1 054 175          FR-A- 2 090 963**

EP 0 907 612 B1

**Description**

La présente invention a pour objet un procédé de préparation d'une silice de précipitation abrasive présentant un diamètre médian d'au moins 10 µm avec une bonne cohésion de grain, susceptible d'être utilisée dans les compositions dentifrices, notamment dans les compositions antitartre ; elle a également pour objet les compositions dentifrices contenant ladite silice.

Les agents abrasifs (notamment la silice) mis en oeuvre dans les compositions dentifrices présentent généralement un diamètre médian de particule inférieur ou égal à 10 µm, moins de 1,5% du poids desdites particules présentant un diamètre supérieur à 44 µm.

Certaines formulations, notamment celles ayant un effet antitartre, peuvent nécessiter la mise en oeuvre d'agents abrasifs de taille supérieure.

Un effet dit "crunchy", à savoir de sensation du grain en bouche, et ce tout au long du brossage, peut être recherché par le consommateur. Un abrasif ne peut permettre d'atteindre cet effet que s'il présente une cohésion suffisante de grain pendant le brossage.

Ce problème peut être résolu par des gels de silice (édifice rigide tridimensionnel continu) de surface spécifique BET généralement supérieure à 300 m2/g. Toutefois le procédé de préparation de ces gels nécessite des étapes de lavage et de filtration longues et coûteuses.

Les silices de précipitation (agglomérats constitués d'entités de particules discrètes liées par des liaisons faibles) sont obtenues par des procédés plus faciles à mettre en oeuvre, mais ne présentent pas un niveau de cohésion de grain suffisant pour l'application recherchée.

La demande de brevet FR-A-1 054 175 décrit la préparation de silices pour caoutchouc selon un procédé par précipitation en presence d'une quantité élevée de sel électrolyte.

La demande de brevet FR-A-2 090 963 décrit la préparation de silices pour caoutchouc selon un procédé par précipitation à un pH de 7 à 10, de préférence 8,5.

La demande de brevet EP-A-520 862 décrit la préparation de silices facilement désagglomérables pour caoutchouc selon un procédé par précipitation à un pH d'au moins 7, avec mise en oeuvre d'un pied de cuve contenant une forte quantité de silicate.

La demande de brevet WO 95/18066 décrit la préparation de silices abrasives pour dentifrice selon un procédé par précipitation à un pH de 8,9 à 9,6.

La demande de brevet EP-A-64 620 décrit la préparation de silices pour filtration de la bière selon un procédé par précipitation dans une large gamme de pH (3-10) et de température (50-95°C).

La Demanderesse a trouvé un procédé de préparation simple par précipitation d'une silice abrasive présentant un diamètre médian d'au moins 10 µm avec une cohésion suffisante de grain.

Un premier objet de l'invention consiste en un procédé de préparation d'une silice de précipitation abrasive présentant

- une surface spécifique BET de l'ordre de 15 à 300 m$^2$/g, de préférence de l'ordre de 20 à 250 m$^2$/g
- une prise d'huile DOP de l'ordre de 40 à 160 ml/100g, de préférence de l'ordre de 50 à 140 ml/100g
- un diamètre médian de particule d'au moins 10µm, généralement de l'ordre de 12 à 30 µm
- un facteur de cohésion de ses particules d'au moins 85% environ pour un diamètre médian de particule de l'ordre de 12 µm à 20 µm

par réaction d'un silicate de métal alcalin M, de rapport SiO$_2$ / M$_2$O de l'ordre de 2 à 4, de préférence de l'ordre de 3 à 3,8 , avec un agent acidifiant, éventuellement mûrissement de la bouillie de silice formée, séparation et séchage de la suspension de silice récupérée et broyage si nécessaire pour obtenir un diamètre médian de particule d'au moins 10µm, généralement de l'ordre de 12 à 30 µm, ledit procédé étant caractérisé en ce que l'opération de formation de la bouillie de silice est réalisée selon les étapes suivantes :

- une première étape consistant à mettre en oeuvre un pied de cuve initial constitué d'eau, d'un sel électrolyte du groupe des métaux alcalins ou alcalino-terreux, éventuellement d'un silicate de métal alcalin et éventuellement d'un agent acidobasique, à une température de l'ordre de 70 à 98°C, de préférence de l'ordre de 80 à 95°C, la quantité d'électrolyte présente étant de l'ordre de 0,1 à 1 mole de sel électrolyte de métal alcalin ou de l'ordre de 10 à 100 mmoles de sel électrolyte de métal alcalino-terreux par litre de pied de cuve et la quantité éventuelle de silicate exprimée en SiO$_2$ étant inférieure à 10g/l;
- une deuxième étape consistant à introduire dans ledit pied de cuve le silicate de métal alcalin en solution aqueuse et l'agent acidifiant, dans des conditions telles que le pH du milieu réactionnel reste sensiblement constant et de l'ordre de 5,5 à 6,7 de préférence de 5,8 à 6,7, lesdits réactifs étant introduits jusqu'à obtention de la concentration désirée en silice dans ledit milieu, le milieu réactionnel étant maintenu à une température de l'ordre de 70 à 98°C, de préférence de l'ordre de 80 à 95°C ;

- et, après un éventuel mûrissement, une troisième étape éventuelle, consistant à acidifier le milieu réactionnel jusqu'à obtenir une bouillie de silice de pH inférieur à 6, de préférence inférieure à 5, tout particulièrement de l'ordre de 4.

Ladite silice présente en outre une surface spécifique CTAB de l'ordre de 10 à 120 $m^2/g$, de préférence de l'ordre de 15 à 100 $m^2/g$.

La surface spécifique BET est déterminée selon la méthode de BRUNAUER-EMMET - TELLER décrite dans "The journal of the American Chemical Society", Vol. 60, page 309, février 1938 et correspondant à la norme ISO 5794/1 (annexe D).

La prise d'huile DOP est déterminée selon la norme ISO 787/5 en mettant en oeuvre du dioctylphtalate.

La cohésion des particules de silice est quantifiée à l'aide d'un test spécifique de cohésion par ultrasonification ; ce test permet d'évaluer l'évolution du diamètre médian $d_{50}$ d'une suspension de silice, par mesure granulométrique avant et après traitement aux ultra-sons.

Selon ce test, la mesure granulométrique (par diffraction laser sur un granulomètre LASER SYMPATEC), est effectuée sur une suspension de silice traitée aux ultra-sons à l'aide d'un sonificateur VIBRACELL BIOBLOCK (puissance de 600W), équipé d'une sonde de diamètre de 19mm, d'une minuterie et d'un convertisseur, selon le mode opératoire suivant :

- préparation de la suspension de silice

On prépare, à l'aide d'un disperseur à hélice RAYNERI une suspension homogène de 15g de silice dans 135g d'eau ; 70g de suspension sont ensuite transvasés dans un flacon en verre de 50ml.

- traitement aux ultra-sons

La sonde, préalablement réglée, est introduite dans le flacon sur une longueur de 4cm , sans contact avec les parois en verre ; la minuterie est programmée avec pulsation sur une durée de 2000 secondes, de manière à obtenir un cycle actif d'ultra-sons de 600 secondes.

Le cycle d'ultra-sons est enclenché après fermeture du flacon.

- contrôle de granulométrie

Après homogénéisation manuelle du flacon fermé, on prélève avec une pipette environ 2ml de suspension homogène, suspension qui est ensuite versée dans la cuve du granulomètre, après ajustement éventuel du niveau d'eau afin d'obtenir une concentration optique de 20% $\pm$ 3%.

Après 30s de dispersion aux ultra-sons de la suspension dans la cuve, on mesure le diamètre médian $d_{50}$ à l'aide d'un dispositif de focale 100mm.

On calcule ensuite le facteur de cohésion de la silice testée ; plus la cohésion de la silice est élevée, plus ce facteur est est élevé.

- facteur de cohésion

Le diamètre médian initial de particule $d_{50i}$ est mésuré sur la suspension homogène de silice avant le traitement aux ultra-sons.

Le diamètre médian final $d_{50f}$ est mésuré sur la suspension homogène de silice après le traitement aux ultrasons.

Le facteur de cohésion FC en (%) est calculé selon l'équation suivante :

$$FC=(d_{50f}/d_{50i}) \times 100$$

Une valeur du FC de 100 correspond à la valeur maximale de cohésion.

La surface spécifique CTAB est la surface externe déterminée selon la norme NFT 45007 (novembre 1987).

Le choix du silicate et de l'agent acidifiant pour réaliser le procédé de l'invention, se fait d'une manière bien connue en soi.

Le silicate de métal alcalin est avantageusement un silicate de sodium ou de potassium. On peut citer tout particulièrement les silicates de sodium.

Ledit silicate est mis en oeuvre sous forme d'une solution aqueuse présentant une concentration exprimée en $SiO_2$, de l'ordre de 150 à 400 g/l, de préférence de l'ordre de 200 à 400 g/l.

On utilise généralement comme agent acidifiant un acide minéral fort tel que l'acide sulfurique, l'acide nitrique, l'acide chlorhydrique ou un acide organique comme l'acide acétique, l'acide formique ou l'acide carbonique. D'une manière préférentielle, il s'agit de l'acide sulfurique. Ce dernier peut être mis en oeuvre sous forme dilué ou concentré, de préférence sous forme d'une solution aqueuse présentant une concentration de l'ordre de 60 à 400 g/l.

Parmi les électrolytes on peut citer notamment le sel du métal du silicate de départ et de l'agent acidifiant, à savoir de préférence le sulfate de sodium.

Un agent acidobasique peut être mis en oeuvre dans le pied de cuve initial pour assurer un pH dudit pied de cuve voisin de celui choisi pour la deuxième étape du procédé, ce pH pouvant être éventuellement ajusté à l'aide d'un acide fort ou d'une base forte. On peut citer comme agent acidobasique, notamment les hydrogénophosphates de métaux alcalins.

Ledit pied de cuve peut en outre renfermer des silicates ; la quantité d'ions silicates éventuels, exprimée en $SiO_2$, est toutefois inférieure à 10 grammes par litre de pied de cuve. De préférence, le pied de cuve ne contient pas d'ions silicates.

Le pied de cuve obtenu est porté à une température de l'ordre 70 à 98°C, de préférence de l'ordre de 80 à 95°C, et maintenu sous agitation.

La deuxième étape consiste à ajouter au pied de cuve maintenu sous forte agitation, la solution de silicate et l'agent acidifiant simultanément.

Les quantités respectives de silicate de métal alcalin et d'agent acidifiant sont choisies de manière à maintenir le pH du milieu réactionnel à une valeur sensiblement constante de l'ordre de 5,5 à 6,7 , de préférence de l'ordre de 5,8 à 6,7, pendant toute l'introduction des deux réactifs.

Ces deux solutions sont introduites en maintenant le milieu à une température de l'ordre de 70 à 98°C, de préférence de l'ordre de 80 à 95°C.

On arrête l'introduction de la solution de silicate lorsque la quantité désirée de silice formée est obtenue. Cette quantité est d'au moins 65 grammes par litre environ, généralement de l'ordre de 65 à 120 grammes par litre, de préférence de l'ordre de 70 à 100 grammes par litre de milieu réactionnel.

La troisième étape éventuelle est réalisée en poursuivant l'addition d'agent acidifiant dans le milieu réactionnel sous agitation, dans les mêmes conditions de température, jusqu'à obtention d'un pH inférieur à 6, de préférence inférieur à 5, tout particulièrement de l'ordre de 4, ce afin d'améliorer les opérations de filtration et de lavage.

Selon une variante de réalisation, le milieu obtenu en fin de deuxième étape après arrêt de l'introduction de la solution de silicate, est laissé mûrir dans les mêmes conditions de température, pendant environ au moins une dizaine de minutes, de préférence de 10 minutes à 2 heures, avant réintroduction de l'agent acidifiant pour réaliser la troisième étape éventuelle.

En fin de troisième étape, après arrêt de l'addition d'agent acidifiant, on laisse éventuellement mûrir le milieu réactionnel, dans les mêmes conditions de température. Cette opération de mûrissement éventuelle peut durer de l'ordre de 10 minutes à 2 heures.

On obtient à l'issue des opérations ci-dessus décrites une bouillie de silice, qui est ensuite séparée (séparation liquide-solide) ; cette opération consiste généralement en une filtration (par exemple à l'aide d'un filtre rotatif sous vide), suivie d'un lavage à l'eau.

La suspension de silice ainsi récupérée (gâteau de filtration) contient au moins 35% environ en masse de matière sèche. Elle est ensuite éventuellement séchée, par exemple à l'aide d'un sécheur convectif (atomiseur, spin-flash, four tunnel ...) ou conductif (four tournant, séchoir à vis, écailleuse, séchoir tambour ...).

La silice en suspension ou sous forme sèche présente un facteur de cohésion de ses particules d'au moins 85% environ, pour un diamètre médian de particule de l'ordre de 12 $\mu$m à 20 $\mu$m.

Celle-ci peut éventuellement être broyée jusqu'à l'obtention du diamètre médian $d_{50}$ de particules désiré, généralement de l'ordre de 10 à 40 $\mu$m, de préférence de l'ordre de 12 à 30 $\mu$m.

La silice présentant une bonne cohésion de grain faisant l'objet de l'invention ou obtenue selon le procédé faisant l'objet de l'invention est particulièrement apte à être utilisée comme agent abrasif dans les compositions dentifrices.

Lorsque la silice se présente sous forme d'une suspension, celle-ci peut être stabilisée par tout moyen connu, notamment à l'aide d'un hydrocolloïde, notamment à l'aide d'un polysaccharide comme la gomme xanthane, la gomme guar, les éthers cellulosiques hydrosolubles ...

La présente invention a également pour objet l'utilisation de la silice faisant l'objet de l'invention ou obtenue selon le procédé de l'invention comme agent abrasif dans les compositions dentifrices, ainsi que les compositions dentifrices comprenant ladite silice. Ladite silice peut être présente dans lesdites compositions dentifrices à raison de l'ordre de 5 à 50% du poids desdites compositions.

Ces compositions peuvent en outre renfermer d'autres ingrédients habituels, en particulier des agents abrasifs minéraux autres, insolubles dans l'eau, des agents épaississants, des humectants ...

Comme agents abrasifs autres, on peut mentionner en particulier les silices abrasives de granulométrie standard (moins de 10μm), le carbonate de calcium, l'alumine hydratée, la bentonite, le silicate d'aluminium, le silicate de zirconium, les métaphosphates et phosphates de sodium, de potassium, de calcium et de magnésium.

La quantité totale de poudre(s) abrasive(s) peut constituer de l'ordre de 5 à 50% du poids de la composition dentaire. Parmi les agents épaississants on peut mentionner tout particulièrement les silices épaississantes en quantité de l'ordre de 1 à 15 % du poids, la gomme xanthane, la gomme guar, les carraghénanes, les dérivés de la cellulose, les alginates, en quantité pouvant aller jusqu'à 5% du poids de ladite composition ....

Parmi les agents humectants on peut citer par exemple le glycérol, le sorbitol, les polyethylène glycols, les polypropylène glycols, le xylitol, en quantité de l'ordre de 2 à 85%, de préférence de l'ordre de 3 à 55% du poids de composition dentifrice exprimé en sec.

Ces compositions dentifrices peuvent en outre comporter des agents tensio-actifs, des agents détergents, des colorants, des anti-bactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym ...)...

Les exemples suivants sont donnés à titre illustratif.

### Exemple 1 comparatif

On introduit dans un réacteur de 25 litres,

- 1110g d'une solution aqueuse de silicate de sodium de rapport $SiO_2/ Na_2O$ de 3,4 contenant 236g/l de $SiO_2$
- 1,8 litres d'eau
- 58,6g de sulfate de sodium.

La température est amenée à 90°C sous forte agitation.

On ajoute ensuite 856g d'acide sulfurique à 80g/l, pour amener le pH à 9,2.

On introduit ensuite simultanément une solution aqueuse de silicate de sodium de rapport $SiO_2/Na_2O$ de 3,4 à 236g/l de $SiO_2$ et une solution aqueuse à 80g/l d'acide sulfurique, à un pH constant de 9,2 de façon à atteindre une concentration de 76g de $SiO_2$ par litre de suspension.

L'addition d'acide est prolongée jusqu'à obtention d'un pH de 4.

Le produit est ensuite filtré, lavé et séché par atomisation.

### Exemple 2 comparatif

On introduit 3 litres d'eau dans un réacteur de 25 litres.

La température est amenée à 80°C sous forte agitation.

On ajoute simultanément une solution aqueuse de silicate de sodium de rapport $SiO_2/Na_2O$ de 3,5 contenant 230g/l de $SiO_2$ et une solution aqueuse à 80g/l d'acide sulfurique, pendant 80 minutes à un pH constant de 6, de façon à atteindre une concentration de 84g de $SiO_2$ par litre de suspension.

L'addition d'acide est prolongée jusqu'à obtention d'un pH de 4.

Le produit est ensuite filtré, lavé et séché par atomisation.

### Exemple 3

On introduit 3 litres d'eau et 156g de sulfate de sodium dans un réacteur de 25 litres.

La température est amenée à 80°C sous forte agitation.

On ajoute simultanément une solution aqueuse de silicate de sodium de rapport $SiO_2/Na_2O$ de 3,5 contenant 230g/l de $SiO_2$ et une solution aqueuse à 80g/l d'acide sulfurique, pendant 80 minutes à un pH constant de 6, de façon à atteindre une concentration de 84g de $SiO_2$ par litre de suspension.

L'addition d'acide est prolongée jusqu'à obtention d'un pH de 4.

Le produit est ensuite filtré, lavé et séché par atomisation.

### Exemple 4

On introduit 3 litres d'eau et 156g de sulfate de sodium dans un réacteur de 25 litres.

La température est amenée à 70°C sous forte agitation.

On ajoute simultanément une solution aqueuse de silicate de sodium de rapport $SiO_2/Na_2O$ de 3,5 contenant

230g/l de SiO$_2$ et une solution aqueuse à 80g/l d'acide sulfurique, pendant 80 minutes à un pH constant de 6, de façon à atteindre une concentration de 84g de SiO$_2$ par litre de suspension.

L'addition d'acide est prolongée jusqu'à obtention d'un pH de 4.

Le produit est ensuite filtré, lavé et séché par atomisation.

## Exemple 5

On introduit 3 litres d'eau et 156g de sulfate de sodium dans un réacteur de 25 litres.

La température est amenée à 90°C sous forte agitation.

On ajoute simultanément une solution aqueuse de silicate de sodium de rapport SiO$_2$/Na$_2$O de 3,5 contenant 230g/l de SiO$_2$ et une solution aqueuse à 80g/l d'acide sulfurique, pendant 80 minutes à un pH constant de 6, de façon à atteindre une concentration de 84g de SiO$_2$ par litre de suspension.

L'addition d'acide est prolongée jusqu'à obtention d'un pH de 4.

Le produit est ensuite filtré, lavé et séché par atomisation.

## Exemple 6

On introduit 3 litres d'eau et 156g de sulfate de sodium dans un réacteur de 25 litres.

La température est amenée à 85°C sous forte agitation.

On ajoute simultanément une solution aqueuse de silicate de sodium de rapport SiO$_2$/Na$_2$O de 3,5 contenant 230g/l de SiO$_2$ et une solution aqueuse à 80g/l d'acide sulfurique, pendant 80 minutes à un pH constant de 6, de façon à atteindre une concentration de 84g de SiO$_2$ par litre de suspension.

L'addition d'acide est prolongée jusqu'à obtention d'un pH de 4.

Le produit est ensuite filtré, lavé et séché par atomisation.

## Exemples 7 et 8

On introduit 3 litres d'eau, 96g de sulfate de sodium et 24g de monohydrogénophosphate de sodium dans un réacteur de 25 litres. Le pH du milieu est de 8,5.

La température est amenée à 85°C sous forte agitation.

Le pH du milieu est amené à 6,2 par addition d'acide sulfurique.

On ajoute simultanément une solution aqueuse de silicate de sodium de rapport SiO$_2$/Na$_2$O de 3,5 contenant 230g/l de SiO$_2$ et une solution aqueuse à 80g/l d'acide sulfurique, pendant 80 minutes à un pH constant de 6,2 , de façon à atteindre une concentration de 84g de SiO$_2$ par litre de suspension.

L'addition d'acide est prolongée jusqu'à obtention d'un pH de 4.

Le produit est ensuite filtré et lavé.

On obtient une suspension contenant 49% de matière sèche **(exemple 7)**.

Cette suspension est ensuite séchée par atomisation **(exemple 8)**.

Les caractéristiques des silices obtenues aux exemples 1 à 8 figurent au tableau 1 suivant.

| exemple | BET (m$^2$/g) | CTAB (m$^2$/g) | prise DOP ml/g | d$_{50i}$ $\mu$m | d$_{50f}$ $\mu$m | FC % |
|---|---|---|---|---|---|---|
| **1** | 40 | 25 | 70 | 14,7 | 11,2 | 76 |
| **2** | 222 | - | 87 | 14,5 | 11,8 | 81 |
| **3** | 80 | 51 | 80 | 15,1 | 14,3 | 95 |
| **4** | 250 | 89 | 132 | 16,3 | 14,8 | 90 |
| **5** | 40 | 26 | 70 | 15,4 | 15,4 | 100 |
| **6** | 60 | 39 | 81 | 17,2 | 16 | 93 |
| **7 (suspension)** | - | - | - | 14,9 | 13,26 | 89 |
| **8** | 71 | 44 | 76 | 14,6 | 13 | 89 |

On constate que les produits préparés selon le procédé de l'invention, de d$_{50i}$ de l'ordre de 15$\mu$m présentent un facteur de cohésion FC d'au moins 85%.

**Exemple 9**

La silice obtenue à l'un quelconque des exemples 3 à 6 ou 8 est mise en oeuvre pour l'obtention de la composition dentifrice suivante.

| | |
|---|---|
| sorbitol (solution à 30%) | 47% |
| silice des exemples 3 à 6 ou 8 | 19% |
| silice épaississante | 3% |
| pyrophosphate tetrasodique | 1% |
| pyrophosphate tetrapotassique | 4% |
| dioxyde de titane | 0,5% |
| gomme xanthane | 0,8% |
| saccharinate de sodium | 0,2% |
| benzoate de sodium | 0,3% |
| fluorure de sodium | 0,22% |
| laurylsulfate de sodium | 1,4% |
| arôme | 1% |
| eau désionisée | qsp 100% |

**Revendications**

1.  Procédé de préparation d'une silice de précipitation abrasive présentant

    -   une surface spécifique BET de l'ordre de 15 à 300 $m^2/g$, de préférence de l'ordre de 20 à 250 $m^2/g$
    -   une prise d'huile DOP de l'ordre de 40 à 160 ml/100g, de préférence de l'ordre de 50 à 140 ml/100g
    -   un diamètre médian de particule d'au moins 10μm, généralement de l'ordre de 12 à 30 μm
    -   un facteur de cohésion de ses particules d'au moins 85% environ pour un diamètre médian de particule de l'ordre de 12 μm à 20 μm

    par réaction d'un silicate de métal alcalin M, de rapport $SiO_2$ / $M_2O$ de l'ordre de 2 à 4, de préférence de l'ordre de 3 à 3,8 , avec un agent acidifiant, éventuellement mûrissement de la bouillie de silice formée, séparation et séchage de la suspension de silice récupérée et broyage si nécessaire pour obtenir un diamètre médian de particule d'au moins 10μm, généralement de l'ordre de 12 à 30 μm, ledit procédé étant **caractérisé en ce que** l'opération de formation de la bouillie de silice est réalisée selon les étapes suivantes :

    -   une première étape consistant à mettre en oeuvre un pied de cuve initial constitué d'eau, d'un sel électrolyte du groupe des métaux alcalins ou alcalino-terreux, éventuellement d'un silicate de métal alcalin et éventuellement d'un agent acidobasique, à une température de l'ordre de 70 à 98°C, de préférence de l'ordre de 80 à 95°C, la quantité d'électrolyte présente étant de l'ordre de 0,1 à 1 mole de sel électrolyte de métal alcalin ou de l'ordre de 10 à 100 mmoles de sel électrolyte de métal alcalino-terreux par litre de pied de cuve et la quantité éventuelle de silicate exprimée en $SiO_2$ étant inférieure à 10g/l;
    -   une deuxième étape consistant à introduire dans ledit pied de cuve le silicate de métal alcalin en solution aqueuse et l'agent acidifiant, dans des conditions telles que le pH du milieu réactionnel reste sensiblement constant et de l'ordre de 5,5 à 6,7, de préférence de 5,8 à 6,7, lesdits réactifs étant introduits jusqu'à obtention de la concentration désirée en silice dans ledit milieu, le milieu réactionnel étant maintenu à une température de l'ordre de 70 à 98°C, de préférence de l'ordre de 80 à 95°C;
    -   et, après un éventuel mûrissement, une troisième étape éventuelle, consistant à acidifier le milieu réactionnel jusqu'à obtenir une bouillie de silice de pH inférieur à 6, de préférence inférieur à 5, tout particulièrement de l'ordre de 4.

2.  Procédé selon la revendication 1) **caractérisé en ce que** ladite silice présente une surface spécifique CTAB de l'ordre de 10 à 120 $m^2/g$, de préférence de l'ordre de 15 à 100 $m^2/g$.

3.  Procédé selon la revendication 1) ou 2) **caractérisé en ce que** le silicate de métal alcalin est un silicate de sodium ou de potassium.

**4.** Procédé selon l'une quelconque des revendications 1) à 3), **caractérisé en ce que** le silicate de métal alcalin est un silicate de sodium mis en oeuvre sous forme d'une solution aqueuse présentant une concentration exprimée en SiO$_2$, de l'ordre de 150 à 400 g/l, de préférence de l'ordre de 200 à 400 g/l.

**5.** Procédé selon l'une quelconque des revendications 1) à 4), **caractérisé en ce que** l'agent acidifiant est un acide minéral ou organique.

**6.** Procédé selon la revendication 5), **caractérisé en ce que** l'agent acidifiant est l'acide sulfurique, l'acide nitrique, l'acide chlorhydrique, l'acide acétique, l'acide formique ou l'acide carbonique.

**7.** Procédé selon la revendication 6), **caractérisé en ce que** l'agent acidifiant est de l'acide sulfurique mis en oeuvre sous forme d'une solution aqueuse présentant une concentration de l'ordre de 60 à 400 g/l.

**8.** Procédé selon l'une quelconque des revendications 1) à 7), **caractérisé en ce que** l'électrolyte est un sel du métal du silicate de départ et de l'agent acidifiant, de préférence du sulfate de sodium.

**9.** Procédé selon l'une quelconque des revendications 1) à 8), **caractérisé en ce que** l'agent acidobasique est un hydrogénophosphate de métal alcalin.

**10.** Procédé selon l'une quelconque des revendications 1) à 9), **caractérisé en ce que** la deuxième étape consiste à ajouter au pied de cuve maintenu sous agitation, la solution de silicate et l'agent acidifiant simultanément, selon des quantités respectives de silicate de métal alcalin et d'agent acidifiant choisies de manière à maintenir le pH du milieu réactionnel à une valeur sensiblement constante de l'ordre de 5,5 à 6,7, de préférence de l'ordre de 5,8 à 6,7, pendant toute l'introduction des deux réactifs.

**11.** Procédé selon l'une quelconque des revendications 1) à 10), **caractérisé en ce que** la troisième étape est réalisée en poursuivant l'addition d'agent acidifiant dans le milieu réactionnel sous agitation, jusqu'à obtention d'un pH inférieur à 6, de préférence inférieur à 5, tout particulièrement de l'ordre de 4.

**12.** Procédé selon l'une quelconque des revendications 1) à 11), **caractérisé en ce que** le milieu obtenu en fin de deuxième étape après arrêt de l'introduction de la solution de silicate, est laissé mûrir pendant au moins une dizaine de minutes, de préférence pendant 10 minutes à 2 heures.

**13.** Procédé selon l'une quelconque des revendications 1) à 12), **caractérisé en ce qu'**en fin de troisième étape, après arrêt de l'addition d'agent acidifiant, on laisse mûrir le milieu réactionnel, pendant environ 10 minutes à 2 heures.

**14.** Procédé selon l'une quelconque des revendications 1) à 13), **caractérisé en ce que** l'opération de formation de la bouillie de silice est réalisée à une température de l'ordre 70 à 98°C, de préférence de l'ordre de 80 à 95°C.

**15.** Utilisation de la silice obtenue selon le procédé faisant l'objet de l'une quelconque des revendications 1) à 14), comme agent abrasif dans les compositions dentifrices.

**16.** Compositions dentifrices comprenant de l'ordre de 5 à 50% de leur poids de silice obtenue selon le procédé faisant l'objet de l'une quelconque des revendications 1) à 14).

**17.** Compositions dentifrices selon la revendication 16), **caractérisées en ce qu'**elles comprennnent en outre au moins un agent ingrédient habituel choisi parmi les agents abrasifs autres, les agents épaississants, les humectants, les agents tensio-actifs, les agents détergents, les colorants, les agents anti-bactériens, les dérivés fluorés, les opacifiants, les arômes, les édulcorants, les agents anti-tartre, anti-plaque, les agents de blanchiment, le bicarbonate de sodium, les antiseptiques, les enzymes, les extraits naturels.

**Claims**

**1.** Process for preparing an abrasive precipitation silica with

- a BET specific surface of about 15 to 300 m$^2$/g, preferably of about 20 to 250 m$^2$/g

- a DOP oil uptake of about 40 to 160 ml/100 g, preferably of about 50 to 140 ml/100 g
- a median particle diameter of at least 10 μm, generally of about 12 to 30 μm
- a particle cohesion factor of at least 85% approximately for a median particle diameter of about 12 μm to 20 μm

by reacting an alkali metal M silicate, with an $SiO_2/M_2O$ ratio of about 2 to 4, preferably of about 3 to 3.8, with an acidifying agent, optional maturation of the silica mash formed, separation and drying of the silica suspension recovered and if necessary grinding so as to obtain a median particle diameter of at least 10 μm, generally of about 12 to 30 μm, the said process being **characterized in that** the operation for forming the silica mash is carried out according to the following steps:

- a first step consisting in using an initial stock . solution consisting of water, an electrolytic salt from the group of alkali metals or alkaline-earth metals optionally an alkali metal silcate and optionally an acidobasic agent, at a temperature of about 70 to 98°C, preferably of about 80 to 95°C, the amount of electrolyte present being about 0.1 to 1 mol of alkalimetal electrolytic salt or of about 10 to 100 mmol of alkaline-earth-metal electrolytic salt per litre of stock solution and the optional amount of silicate expressed as $SiO_2$ being less than 10 g/l;
- a second step consisting in introducing into the said stock solution the alkali metal silicate as an aqueous solution and the acidifying agent, under conditions such that the pH of the reaction medium remains more or less constant and at a value of about 5.5 to 6.7, preferably of about 5.8 to 6.7, the said reagents being introduced until the desired silica concentration is obtained in the said medium, the reaction medium being maintained at a temperature of about 70 to 98°C, preferably of about 80 to 95°C;
- and, after optional maturation, a third optional step consisting in acidifying the reaction medium until a silica mash with a pH of less than 6, preferably less than 5, most particularly of about 4, is obtained.

2. Process according to claim 1, **characterized in that** the said silica has a CTAB specific surface of about 10 to 120 $m^2/g$, preferably of about 15 to 100 $m^2/g$

3. Process according to claim 1 or 2, **characterized in that** the alkali metal silicate is a sodium or potassium silicate.

4. Process according to any one of claims 1 to 3, **characterized in that** the alkali metal silicate is a sodium silicate used in the form of an aqueous solution with a concentration, expressed as $SiO_2$, of about 150 to 400 g/l, preferably of about 200 to 400 g/l.

5. Process according to any one of claims 1 to 4, **characterized in that** the acidifying agent is an inorganic or organic acid.

6. Process according to claim 5, **characterized in that** the acidifying agent is sulphuric acid, nitric acid, hydrochloric acid, acetic acid, formic acid or carbonic acid.

7. Process according to claim 6, **characterized in that** the acidifying agent is sulphuric acid, which is used in the form of an aqueous solution with a concentration of about 60 to 400 g/l.

8. Process according to any one of claims 1 to 7, **characterized in that** the electrolyte is a metal salt of the starting silicate and of the acidifying agent, preferably sodium sulphate.

9. Process according to any one of claims 1 to 8, **characterized in that** the acidobasic agent is an' alkali metal hydrogen phosphate.

10. Process according to any one of claims 1 to 9, **characterized in that** the second step consists in adding the silicate solution and the acidifying agent simultaneously to the stock solution which is kept stirring, the addition being in respective amounts of alkali metal silicate and of acidifying agent which are chosen so as to keep the pH of the reaction medium at a more or less constant value of about 5.5 to 6.7, preferably of about 5.8 to 6.7, throughout the introduction of the two reagents.

11. Process according to any one of claims 1 to 10, **characterized in that** the third step is carried out by adding the acidifying agent to the reaction medium with stirring, until a pH of less than 6, preferably less than 5 and most particularly of about 4, is obtained.

12. Process according to any one of claims 1 to 11, **characterized in that** the medium obtained at the end of the second step, after stopping the introduction of the silicate solution, is left to mature for at least about ten minutes, preferably for 10 minutes to 2 hours.

13. Process according to any one of claims 1 to 12, **characterized in that** at the end of the third step, after stopping the addition of the acidifying agent, the reaction medium is left to mature for about 10 minutes to 2 hours.

14. Process according to any one of claims 1 to.13, **characterized in that** the operation for formation of the. silica mash is carried out at a temperature of about 70 to 98°C, preferably of about 80 to 95°C.

15. Use of the silica which is obtained according to the process which forms the subject-of any one of claims 1 to 14, as an abrasive agent in toothpaste compositions.

16. Toothpaste compositions comprising about 5 to 50% of their weight of silica obtained according to the process which forms the subject of any one of claims 1 to 14.

17. Toothpaste compositions according to claim 16, **characterized in that** they also comprise at least one common ingredient chosen from other abrasive agents, thickeners, wetting agents, surfactants, detergents, dyes, antibacterial agents, fluoro derivatives, opacifiers, flavourings, sweeteners, anti-tartar agents, anti-plaque agents, bleaching agents, sodium bicarbonate, antiseptics, enzymes and natural extracts.


**Patentansprüche**

1. Verfahren zur Herstellung eines abrasiven Fällungs-Siliciumdioxids, das aufweist

   - eine spezifische BET-Oberfläche in der Größenordnung von 15 bis 300 m$^2$/g, vorzugsweise in der Größenordnung von 20 bis 250 m$^2$/g
   - eine Ölaufnahme DOP in der Größenordnung von 40 bis 160 ml/100 g, vorzugsweise in der Größenordnung von 50 bis 140 ml/100 g
   - einen mittleren Teilchendurchmesser von wenigstens 10 μm, im allgemeinen in der Größenordnung von 12 bis 30 μm
   - einen Kohäsionsfaktor dieser Teilchen von wenigstens etwa 85% für einen mittleren Teilchendurchmesser in der Größenordnung von 12 μm bis 20 μm

   durch Umsetzen eines Silikats eines Alkalimetalls M mit einem Verhältnis SiO$_2$/M$_2$O in der Größenordnung von 2 bis 4, vorzugsweise in der Größenordnung von 3 bis 3,8, mit einem Ansäuerungsmittel, gegebenenfalls unter Alterung der gebildeten Siliciumdioxid-Aufschlämmung, Abtrennung und Trocknen der gewonnenen Siliciumdioxidsuspension und, wenn nötig, Zerkleinern, um zu einem mittleren Teilchendurchmesser von wenigstens 10 μm, im allgemeinen in der Größenordnung von 12 bis 30 μm, zu gelangen, wobei dieses Verfahren **dadurch gekennzeichnet ist, daß** die Maßnahme der Bildung der Siliciumdioxidaufschlämmung entsprechend den folgenden Stufen durchgeführt wird:

   - eine erste Stufe, bestehend in dem Einsatz eines Ausgangs-Ansatzes, der aus Wasser, einem Elektrolytsalz der Gruppe der Alkali- oder Erdalkalimetalle, gegebenenfalls einem Alkalimetallsilikat und gegebenenfalls einem Säure-Basenmittel besteht, bei einer Temperatur in der Größenordnung von 70 bis 98°C, vorzugsweise in der Größenordnung von 80 bis 95°C, wobei die anwesende Elektrolytmenge in der Größenordnung von 0,1 bis 1 Mol des Alkalimetallelekrolytsalzes oder in der Größenordnung von 10 bis 100 mMol des Erdalkimetallelektrolytsalzes je Liter Ansatz beträgt und die etwaige Menge an Silikat, ausgedrückt in SiO$_2$, geringer als 10 g/l ist;
   - eine zweite Stufe, bestehend in dem Einbringen in besagten Ansatz des Alkalimetallsilikats in wässriger Lösung und des Ansäuerungsmittels unter derartigen Bedingungen, daß der pH des Reaktionsmilieus im wesentlichen konstant bleibt und in der Größenordnung von 5,5 bis 6,7, vorzugsweise 5,8 bis 6,7, liegt, wobei besagte Reagenzien bis zur Erzielung der gewünschten Konzentration an Siliciumdioxid in diesem Milieu eingebracht werden, wobei das Reaktionsmilieu bei einer Temperatur in der Größenordnung von 70 bis 98°C, vorzugsweise in der Größenordnung von 80 bis 95°C, gehalten wird;
   - und nach einer etwaigen Alterung eine etwaige dritte Stufe, bestehend in der Ansäuerung des Reaktionsmilieus bis zur Erzielung einer Aufschlämmung von Siliciumdioxid mit einem pH von niedriger als 6, vorzugsweise niedriger als 5, insbesondere in der Größenordnung von 4.

**2.** Verfahren gemäß Anspruch 1, **dadurch gekennzeichnet, daß** das Siliciumdioxid eine spezifische CTAB Oberfläche in der Größenordnung von 10 bis 120 m$^2$/g, vorzugsweise in der Größenordnung von 15 bis 100 m$^2$/g, besitzt.

**3.** Verfahren gemäß Anspruch 1 oder 2, **dadurch gekennzeichnet, daß** das Alkalimetallsilikat ein Silikat von Natrium oder Kalium ist.

**4.** Verfahren gemäß einem der Ansprüche 1 bis 3, **dadurch gekennzeichnet, daß** das Alkalimetallsilikat ein Silikat von Natrium ist, das in Form einer wässrigen Lösung eingesetzt wird, die eine Konzentration, ausgedrückt in SiO$_2$, in der Größenordnung von 150 bis 400 g/l, vorzugsweise in der Größenordnung von 200 bis 400 g/l, besitzt.

**5.** Verfahren gemäß einem der Ansprüche 1 bis 4, **dadurch gekennzeichnet, daß** das Ansäuerungsmittel eine Mineralsäure oder organische Säure ist.

**6.** Verfahren gemäß Anspruch 5, **dadurch gekennzeichnet, daß** das Ansäuerungsmittel Schwefelsäure, Salpetersäure, Chlorwasserstoffsäure, Essigsäure, Ameisensäure oder Kohlensäure ist.

**7.** Verfahren gemäß Anspruch 6, **dadurch gekennzeichnet, daß** das Ansäuerungsmittel Schwefelsäure ist, die in Form einer wässrigen Lösung eingesetzt wird, welche eine Konzentration in der Größenordnung von 60 bis 400 g/l aufweist.

**8.** Verfahren gemäß einem der Ansprüche 1 bis 7, **dadurch gekennzeichnet, daß** der Elektrolyt ein Ausgangs-Metallsilikatsalz ist und das Ansäuerungsmittel vorzugsweise Natriumsulfat ist.

**9.** Verfahren gemäß einem der Ansprüche 1 bis 8, **dadurch gekennzeichnet, daß** das Säure-Basen-Mittel ein Alkalimetallhydrogenphosphat ist.

**10.** Verfahren gemäß einem der Ansprüche 1 bis 9, **dadurch gekennzeichnet, daß** die zweite Stufe darin besteht, dem unter Rühren gehaltenen Ansatz, die Lösung des Silikats und des Ansäuerungsmittels gleichzeitig entsprechend den jeweiligen Mengen von Alkalimetallsilikat und Ansäuerungsmittel zuzusetzen, derart, daß der pH des Reaktionsmilieus bei einem im wesentlichen konstanten Wert in der Größenordnung von 5,5 bis 6,7, vorzugsweise in der Größenordnung von 5,8 bis 6,7, während der gesamten Einbringung dieser beiden Reagenzien gehalten wird.

**11.** Verfahren gemäß einem der Ansprüche 1 bis 10, **dadurch gekennzeichnet, daß** die dritte Stufe durchgeführt wird, indem man die Zugabe des Ansäuerungsmittels in das Reaktionsmilieu unter Rühren bis zur Erzielung eines pH von geringer als 6, vorzugsweise geringer als 5, insbesondere in der Größenordnung von 4, vornimmt.

**12.** Verfahren gemäß einem der Ansprüche 1 bis 11, **dadurch gekennzeichnet, daß** man das am Ende der zweiten Stufe nach dem Abbrechen des Einbringens der Silikatlösung erhaltene Milieu während zumindest etwa 10 Minuten bis zu 2 Stunden altern läßt.

**13.** Verfahren gemäß einem der Ansprüche 1 bis 12, **dadurch gekennzeichnet, daß** man am Ende der dritten Stufe nach Abbrechen der Zugabe des Ansäuerungsmittels das Reaktionsmilieu während etwa 10 Minuten bis zu 2 Stunden altern läßt.

**14.** Verfahren gemäß einem der Ansprche 1 bis 13, **dadurch gekennzeichnet, daß** die Maßnahme der Bildung der Siliciumdioxidaufschlämmung bei einer Temperatur in der Größenordnung von 70 bis 98°C, vorzugsweise in der Größenordnung von 80 bis 95°C, durchgeführt wird.

**15.** Verwendung des Siliciumdioxids, erhalten nach dem Verfahren gemäß einem der Ansprüche 1 bis 14, als Schleifmittel in Zahnpasten.

**16.** Zahnpastazusammensetzungen umfassend in der Größenordnung von 5-50 Gew.-% des nach dem Verfahren gemäß einem der Ansprüche 1 bis 14 erhaltenen Siliciumdioxids.

**17.** Zahnpastazusammensetzungen gemäß Anspruch 16, **dadurch gekennzeichnet, daß** sie außerdem wenigstens einen üblichen Bestandteil umfassen, ausgewählt unter weiteren Schleifmitteln, Verdickungsmitteln, Befeuchtungsmitteln, oberflächenaktiven Mitteln, Detergenzien, färbenden Bestandteilen, antibakteriellen Mitteln, Fluorderivaten, Trübungsmitteln, Aromastoffen, Süßungsmitteln, dem Zahnbelag entgegenwirkenden Mitteln, Antiplaquemitteln, Weißungsmitteln, Natriumbicarbonat, antiseptischen Mitteln, Enzymen, natürlichen Extrakten.